# EUROPEAN PATENT APPLICATION

(11) **EP 2 966 443 A1**
(43) Date of publication of application: **13.01.2016**
(21) Application number: 15175728.3
(22) Date of filing: 07.07.2015
(51) Int. Cl.: G01N 33/28, G01N 21/31, G01N 21/85

(54) **ANALYSIS OF FUEL TANK CONTENTS**

(30) Priority: 10.07.2014 GB 201412325
(71) Applicant: Airbus Operations Limited, Bristol BS34 7PA (GB)
(72) Inventor: CIPULLO Alessio, Bristol BS34 7PA (GB); KILVINGTON David, Bristol BS34 7PA (GB); TICHBORNE Franklin, Bristol BS34 7PA (GB)
(74) Representative: Flaxman, Andrew James

(57) **Abstract**

A probe (118) is provided for use in determining at least one characteristic of contents within a fuel tank. The probe (118) includes a plurality of analysis elements (302). Each analysis element (302) has an input (308) for inputting light to a sampling region (316) to be analysed and an output (310) for outputting light that has passed through the sampling region (316) from the input (308). A system including a probe (118) and a spectrometer (406) is also provided. The probe (118) may be mounted to a interior wall of an aircraft fuel tank, or to a rib (112,116) within an aircraft wing (104,106).

## Description

### FIELD OF THE INVENTION

The invention relates to determining a characteristic of contents within a fuel tank.

### BACKGROUND OF THE INVENTION

Generally, it is very important to be able to determine how much fuel is in a fuel tank of a vehicle at a particular time. Such a determination is particularly important in aircraft fuel tanks.

It is known that aircraft fuel tanks often include fluids other than aircraft fuel, such as air and water. It is useful to be able to determine, at least approximately, the volume of aircraft fuel within a fuel tank and the volume of any other fluids within the fuel tank. Some existing systems used in aircraft fuel tanks measure the time of flight of ultrasound pulses and/or RF pulses reflected by fuel/air and fuel/water interfaces within the tank. These and other systems require in-tank electronics which can operate poorly in the harsh conditions within the fuel tank, and which need to be shielded from other electromagnetic sources.

### SUMMARY OF THE INVENTION

A first aspect of the invention provides a probe for use in determining at least one characteristic of contents within a fuel tank. The probe comprises at least one analysis element, the or each analysis element having an input for inputting light to a sampling region to be analysed and an output for outputting light that has passed through the sampling region from the input. The light may then be delivered to means, such as a spectrometer, for measuring the absorption spectrum of the light that has passed through the sampling region. The absorption spectrum can provide information on characteristics of the contents of the fuel tank in the sampling region including the type of fluid present (e.g. air, water or fuel), the density of the fluid present, whether any water is present in any other fluid and, if so, how much water is present, whether any contaminants are present in the fluid and, if so, what contaminants are present, and the volume of each of the contents of the fuel tank.

The or each analysis element may comprise an optical fibre, and the input and the output may be formed from ends of the optical fibre.

The or each analysis element may further comprise an optical element for collecting the light received via the input and/or an optical element for collecting the light output by the analysis element. The or each analysis element may further comprise an optical element for collimating the light received via the input and/or an optical element for collimating the light output by the analysis element. At least one of the optical elements may comprise a lens.

The at least one analysis element may comprise a plurality of analysis elements arranged linearly within the fuel tank.

The probe may be configured to be mounted to an interior wall of an aircraft fuel tank and/or to a rib within an aircraft wing.

A second aspect of the invention provides a system for determining at least one characteristic of contents within a fuel tank. The system comprises a probe as described above; and a spectrometer configured to: receive light from the light output of the or each probe, and perform spectral analysis on said received light so as to determine at least one component of the contents within the sampling region.

The system may further comprise a light source for generating light to be delivered to the input of each analysis element. The light source may comprise a broadband light source or a swept narrowband light source.

The system may further comprise a processor for processing the light received from the light output of the or each analysis element.

The system may comprise at least three probes, each probe being as described above.

A third aspect of the invention provides a method of analysing contents of a fuel tank. The method comprises: providing, within a fuel tank, a probe as described above; providing light to the or each analysis element of the probe via the or each input; receiving light from the output of the or each analysis element; and analysing the absorption spectrum of the received light to determine at least one component of the contents of the fuel tank within the sampling region.

A fourth aspect of the invention provides a method of manufacturing apparatus for analysing contents of a fuel tank. The method comprises: providing a former; positioning at least one optical fibre on the former; at least partially encapsulating the at least one optical fibre and the former with a liquid compound; and forming a channel through the at least one optical fibre and the former to create a sampling region.

A fifth aspect of the invention provides a method of manufacturing apparatus for analysing contents of a fuel tank. The method comprises: embedding at least one optical fibre at least partially within a former; and removing a section from the embedded at least one optical fibre to create a sampling region.

It will be appreciated that the features of the various aspects of the invention may be combined with those of other aspects of the invention.

### BRIEF DESCRIPTION OF THE DRAWINGS

Embodiments of the invention will now be described with reference to the accompanying drawings, in which:
Figure 1 is a plan view of an aircraft;
Figure 2 is an isometric view of part of a fuel tank;
Figure 3 is a schematic view of a probe constructed in accordance with an embodiment of the invention;
Figure 4 is a schematic view of a system constructed in accordance with an embodiment of the invention;
Figures 5 and 6 are schematic views of probes constructed in accordance with alternative embodiments of the invention;
Figure 7 is a schematic diagram of a system constructed in accordance with an alternative embodiment of the invention;
Figure 8 is a flow diagram showing the steps of a method of constructing a probe in accordance with an embodiment of the invention; and
Figure 9 is a diagram showing a probe in various stages of manufacture, in accordance with an embodiment of the invention.

### DETAILED DESCRIPTION OF EMBODIMENT(S)

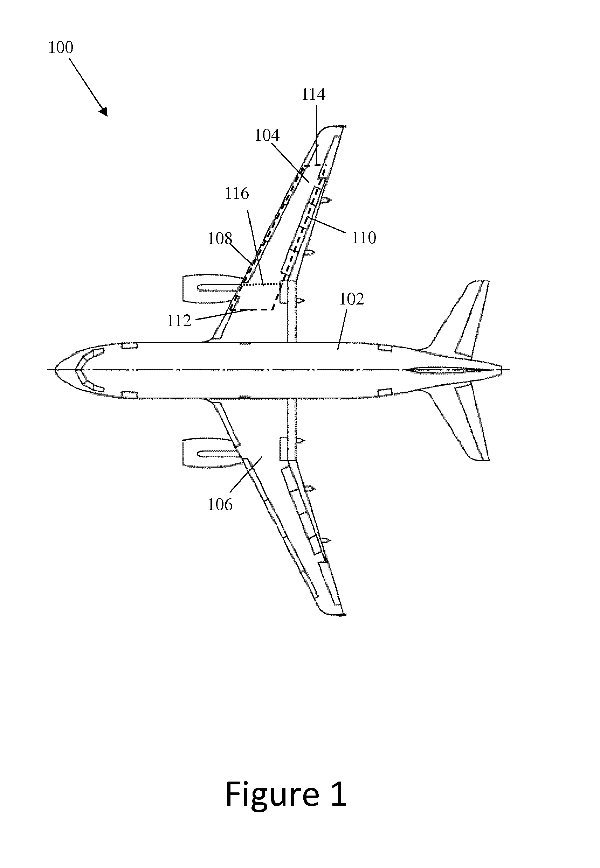

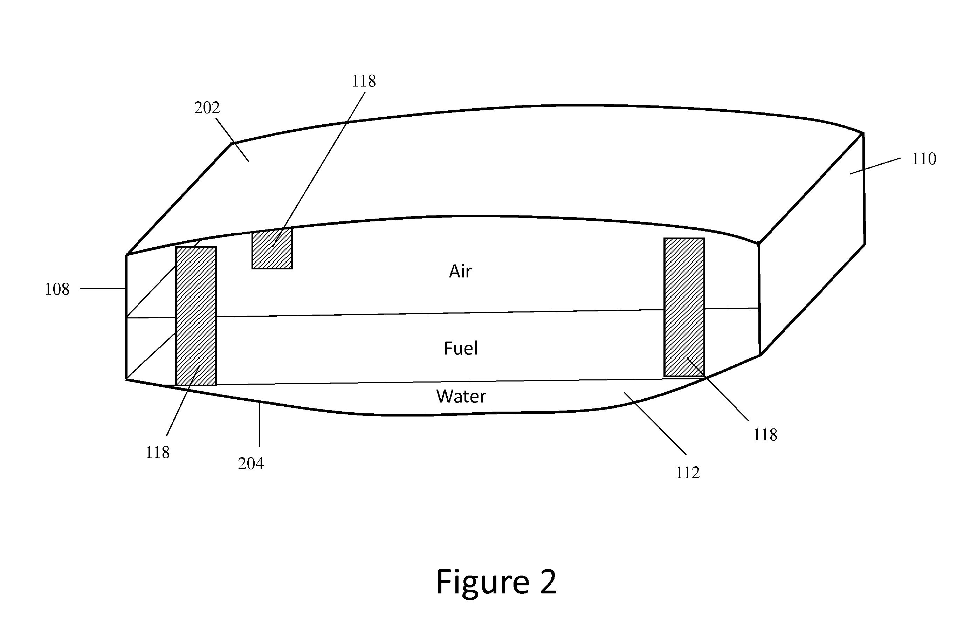

Referring to the drawings, Figure 1 shows, in plan view, a typical aircraft 100 having a fuselage 102 and wings 104, 106. The wing 104 has a wing box which is bounded by front and rear spars 108, 110; inboard and outboard ribs 112, 114; and upper and lower covers 202, 204 (shown in Figure 2) which together form the walls of a fuel tank. The fuel tank is divided into a number of sub-compartments by baffle ribs which allow fuel to flow between the compartments. One of such compartments in shown in Figure 2 bounded by the inboard rib 112 and a baffle rib 116.

The fuel tank is provided with one or more probes 118 which are distributed across its extent. The compartment of the fuel tank shown in Figure 2 includes three probes 118; two probes are mounted on an inner surface of the inbound rib 112 and one probe is mounted on an inner surface of the baffle rib 116. It will be appreciated that, while the embodiment shown in Figure 2 includes three probes 118, each compartment of the fuel tank may include fewer or more probes. Layers of air, fuel and water are shown in the fuel tank as an example. It will, of course, be appreciated that the type and amount of each of the contents in the fuel tank will vary depending on the type of vehicle on which the fuel tank is used. While the invention is described herein with reference to an aircraft fuel tank, it will be appreciated that the invention may be used in a fuel tank of a different type of vehicle, such as an automobile.

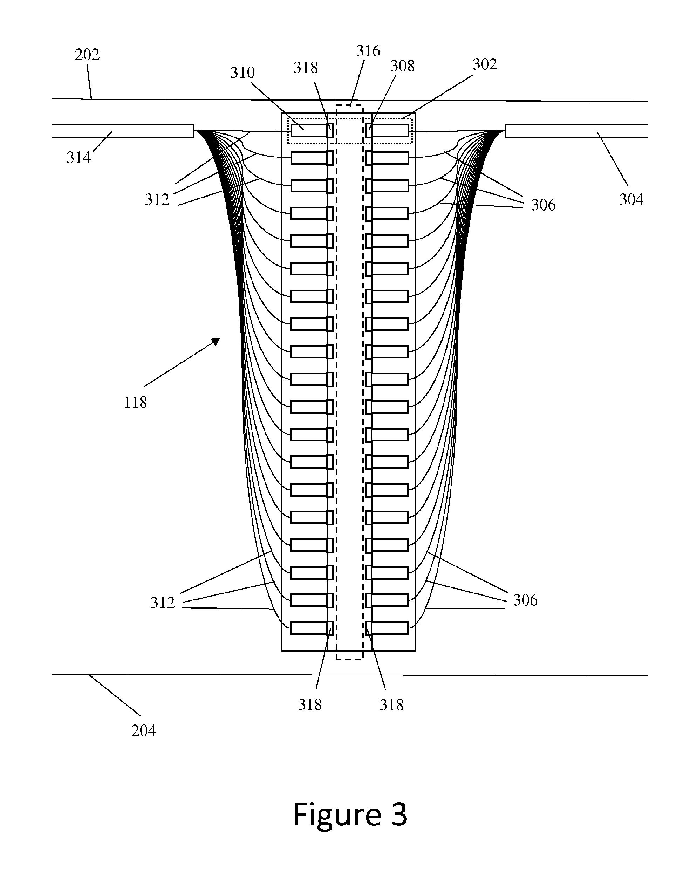

The probe 118 is shown in greater detail in Figure 3. The probe 118 is mounted on the inner surface of a wall of the fuel tank, the upper and lower covers 202, 204 of which are shown in Figure 3. The probe 118 is formed of an elongate mount 300 which extends substantially over the height of the fuel tank wall to which it is mounted, and includes one or more analysis elements 302 spaced equally along the length of the probe. The probe 118 shown in Figure 3 includes nineteen analysis elements but, as will become apparent, in other embodiments, the probe may include more or fewer analysis elements 302. In some embodiments, the probe 118 includes a single analysis element 302.

In this embodiment, an optical fibre bundle 304 provides a means for delivering light to the probe 118, and includes a bundle of individual optical fibres 306, each of which feeds into a respective analysis element 302. In this way, each individual optical fibre 306 provides a means for delivering light to a respective analysis element 302. Each analysis element 302 has an input 308 and an output 310. The input 308 of each analysis element 302 is connected to its respective optical fibre 306 such that each analysis element is able to receive light via its input. The output 310 of each analysis element is connected to one of a plurality of optical fibres 312 which, in turn, are grouped together to form an optical fibre bundle 314.

It will be apparent that, in embodiments of the invention in which the probe 118 includes a single analysis element 302, a single optical fibre 306 will be required to feed light to the input 308 of the analysis element, and a single optical fibre 312 will be required to deliver light from the output 310 of the analysis element. As such, in those embodiments, the optical fibre bundles 304, 314 may be omitted in favour of individual optical fibres.

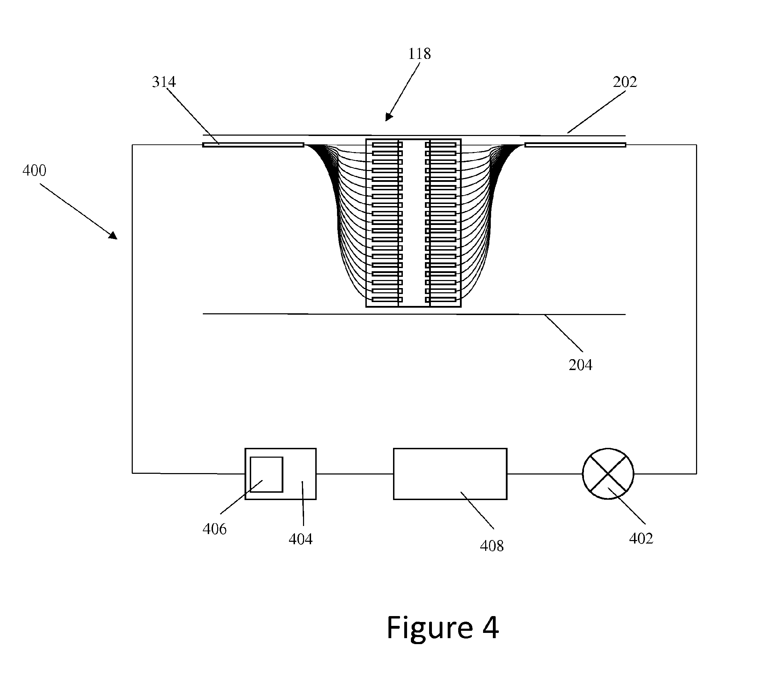

In use, light from a light source 402 (see Figure 4) is delivered to the input 308 of each analysis element 302 by its respective optical fibre 306 of the optical fibre bundle 304. Light travels from the input 308, through a sampling region 316, to the output 310 of the analysis element 302. It will be appreciated that contents of the fuel tank will be present in the sampling region 316 formed between the input 308 and the output 310. The contents of the fuel tank, which may include aircraft fuel, water, air, or a combination of these, will affect the light passing through the sampling region such that, as light travels from the input 308 to the output 310, some of the light will be absorbed by the contents of the fuel tank present in the sampling region 316 between the input and the output. The fluid may also contain contaminants floating in, or dissolved in, the fluid, and such contaminants might also have affect the light passing through the sampling region 316.

The input 308 and/or the output 310 of each analysis element 302 may each be provided with an optical element, such as a lens 318, for collimating the light leaving the input and arriving at the output. In some embodiments, optical elements are not required, or are provided only on the input 308 of each analysis element 302. The use of an optical element such as a lens 318 helps to prevent light from being scattered from an input 308 of one analysis element 302 into an output 310 of another analysis element. In alternative embodiments, one or more light barriers (not shown) may be used to prevent light emitted from an input 308 of one analysis element 302 from entering the output 310 of another analysis element. Alternatively, to achieve the same effect, each analysis element 302 might be encapsulated in its own optical housing (not shown).

After passing through the sampling region 316, the light received by the output 310 of each analysis element 302 is delivered, via the optical fibre 312, into components for analysis as will be discussed with reference to Figure 4.

Figure 4 shows a system 400 which can be used to determine a characteristic of contents within a fuel tank. The system 400 includes the probe 118 shown in Figure 3. Light generated by the light source 402 is delivered to the input 308 of each of the analysis elements 302 via the bundle 304 of individual optical fibres 306. It will be appreciated that, although the probe 118 is mounted to an inner surface of a wall of the fuel tank, the light source 402 may be located outside the fuel tank, and may, for example, be located inside the fuselage of the aircraft. In this embodiment, the light source 402 is a broadband light source, but could alternatively be a swept narrowband light source. Preferably, the light source is configured to emit radiation over a large range of wavelengths, including light in the visible spectral range, infrared (IR) radiation and ultraviolet (UV) radiation. Light emitted by the light source 402 may be pulsed or continuous. In an alternative embodiment, light may be provided by an alternative source, such as a light installed in aircraft, instead of a separate light source 402. For example, for a system installed on an aircraft, a light source used for other purposes on the aircraft might be used to provide light to the system 400. Alternatively, light from outside of the aircraft (e.g. sunlight) may be used instead of light from the light source 402.

Light received by the output 310 of each analysis element 302 is fed through the optical fibre bundle 314 into a photo detector 404, which is located outside the fuel tank. The system 400 also includes a spectrometer 406 which is configured to measure the spectrum of the light or radiation received by each output 310. The photo detector 404 and the spectrometer 406 may be combined in a single unit (as shown in Figure 4) or included individually in the system. The spectrometer 406 separates out the individual spectral components of the light output from each analysis element 302. The photo detector 404, which may include a charge-coupled device (CCD) or some other image capture device, acquires the individual spectral components and converts them into a digital format suitable for analysis. The system 400 may further include an elaboration unit (not shown) to elaborate the spectra obtained by the spectrometer 406. The elaboration unit may calibrate the received spectra using known spectra stored in a memory (not shown) in the system 400 and/or may reduce or remove background noise in signals received from the spectrometer 406 or from the photo detector 404 in order to increase the signal-to-noise ratio (SNR). In alternative embodiments, the spectrometer 406 may be omitted altogether, such that the photo detector 404 receives the light from the output 310 of each analysis element 302 and measures the light intensity without any additional measurements or analysis of the light being performed. A separate spectrum may be measured for each analysis element 302 in the probe 118, each spectrum being individually fed into the spectrometer 406.

The photo detector 404 and/or the spectrometer 406 output signals to a processor 408 which is configured to analyse the measured spectra in order to determine one or more characteristics of the contents of the fuel tank present in the sampling region 316 between the input 308 and the output 310 of each analysis element 302.

In some embodiments, the processor 408 compares each spectrum received from the spectrometer 406 with a collection of known spectra, which may be stored in a storage medium such as a memory (not shown). If a spectrum received from the spectrometer 406 is determined to be substantially the same as a particular spectrum stored in the memory, then the processor determines that the substance to which that spectrum relates is a constituent element of the contents in the particular portion of the sample region 316 between the input 308 and the output 310 of the analysis element 302. For example, if the processor 408 determines that a spectrum received from an output 310 of an analysis element 302 near to the top of the probe 118 contains peaks corresponding to elements found in air, then it might be determined that air is present in the sampling region 316 near to the top of the probe. Similarly, if the processor 408 determines that a spectrum received from an output 310 of an analysis element 302 near to the bottom of the probe 118 contains peaks corresponding to elements found in water, then it might be determined that water is present in the sampling region 316 near to the bottom of the probe.

In this way, the processor 408 is able to determine what element or elements are present in the sampling region 316 at various depths within the fuel tank. More specifically, a determination of the contents of the fuel tank can be made for each analysis element 302 of the probe 118. In this way, the system 400 can determine a profile of the contents of the fuel tank over the height of the fuel tank. By increasing the number of analysis elements 302 in the probe 118, the resolution of the profile can be increased. Similarly, a probe 118 having fewer analysis elements 302 will produce a profile of contents having a lower resolution.

By analysing the measured spectra of the fuel tank contents in the manner described above, the system 400 is able to determine the approximate depth of each different fluid in the fuel tank. Thus, used in a fuel tank containing, for example, a layer of water at the bottom, a layer of aircraft fuel on top of the water layer, and air filling the ullage of the fuel tank above the fuel, the system 400 can be used to determine the approximate depth of each of the water and fuel layers, and the approximate amount of air in the fuel tank.

Typically each compartment of a fuel tank includes three probes 118, each preferably being positioned near to a corner of the fuel tank compartment. With such an arrangement, the processor 408 can, using triangulation, determine the approximate position of a plane of an interface between the air and the fuel and between the fuel and water in the fuel tank. In this way, it is possible to determine the approximate volume of the various contents of the fuel tank.

The contents of a fuel tank are liable to move, or slosh around as the vehicle moves. Such sloshing is particularly prevalent in aircraft fuel tanks. To compensate for the movement of the contents of the fuel tank, the measurements made by each analysis element 302 of the probe 118 may be averaged over a period of time. For example, the processor 408 may average the measurements over a period of ten seconds so that the movement of the water and fuel within the tank does not adversely affect the determination made by the processor.

In addition to the ability to determine the nature of the contents of the fuel tank in the sampling region 316 by analysing the measured absorption spectrum at various depths, it is also possible to determine the density of the contents using the absorption spectrum. Thus, the processor is capable of calculating the density of the fluid present in the sampling region 316 at various depths of within the fuel tank. The calculated densities can also be used to determine the contents by comparing the calculated densities with densities of known fluids which may, for example, be stored in the memory (not shown).

The information determined and/or calculated by the processor 408 may be displayed in real-time to the aircraft pilot or transmitted via transmission means (not shown) to another destination such as, for example, a monitoring station.

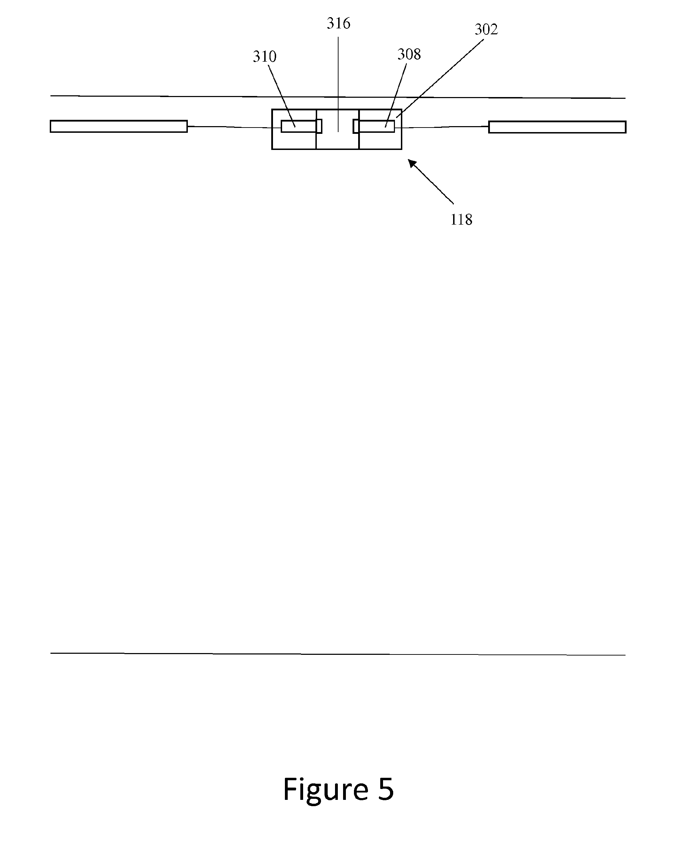

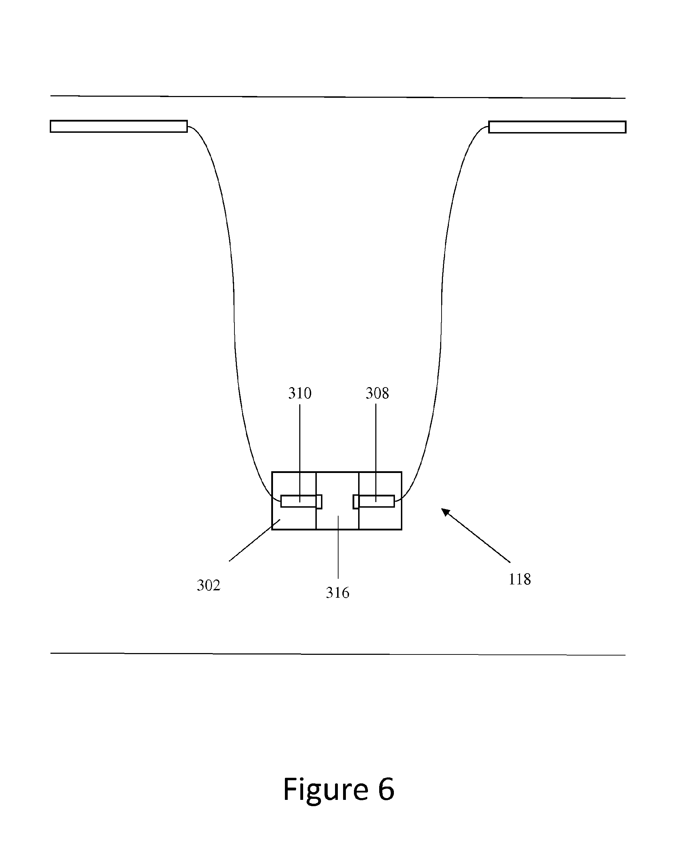

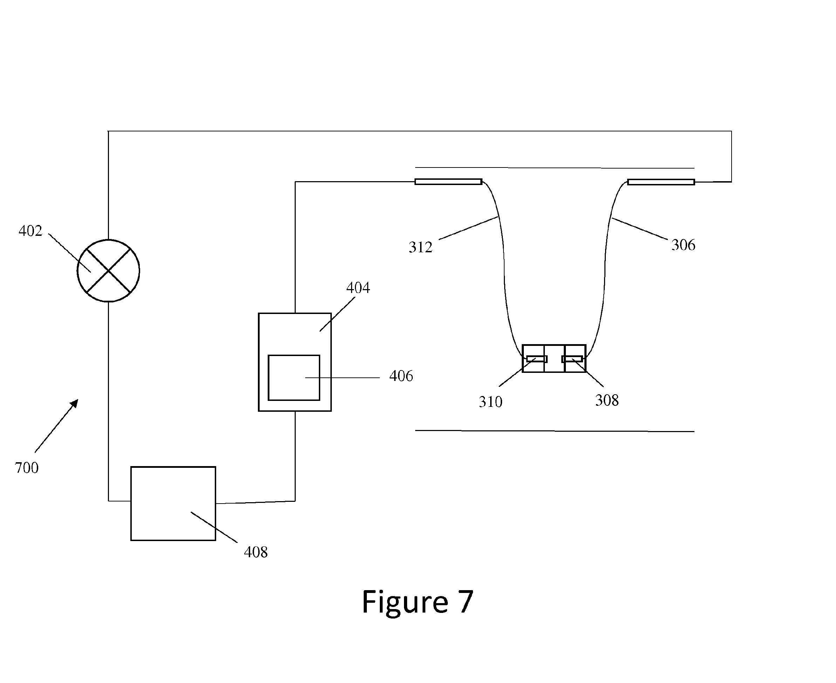

In the embodiments of the invention shown in Figures 5 and 6, the probe 118 includes a single analysis element 302. Figure 7 shows a system 700 which includes the probe 118 of Figure 6. Light is fed to the input 308 via a single optical fibre 306, and light from the output 310 is delivered to the photo detector 404 and/or to the spectrometer 406 via a single optical fibre 312. In the embodiment shown in Figure 5, the analysis element 302 is located near to the top of the fuel tank whereas, in the embodiment shown in Figures 6 and 7, the analysis element 302 is located relatively near to the bottom of the fuel tank. The analysis elements 302 of Figures 5 and 6 function in a manner identical to the analysis elements described above with reference to Figure 3. However, in the embodiment shown in Figure 5, the analysis element 302 may be used to determine when a particular fluid is present in the sampling region 316 between the input 308 and the output 310. For example, the embodiment of Figure 5 may be used to determine when aircraft fuel is at the level of the analysis element 302. Such an arrangement may be useful when filling the fuel tank with fuel; when the analysis element 302 detects aircraft fuel in the sampling region 316, then a signal may be generated to prevent more fuel from being poured into the fuel tank, thereby preventing the fuel overflowing out of the fuel tank. In a similar manner, the probe 118 of Figure 6 may be incorporated into a system such as that shown in Figure 7, wherein the probe may be used to determine when air is detected at the level of the analysis element 302. Such an arrangement may be useful to determine when the amount of fuel in the fuel tank has fallen below a predetermined level - in this case, the level of the analysis element. When the analysis element 302 detects air in the sampling region 316, then a signal may by generated to warn the pilot that the amount of fuel in the fuel tank is below a particular amount.

As is noted above, in use, the probe is located within a fuel tank. Any components requiring electronics can be located outside of the fuel tank and, as such, the system can operate with no electronics inside the fuel tank.

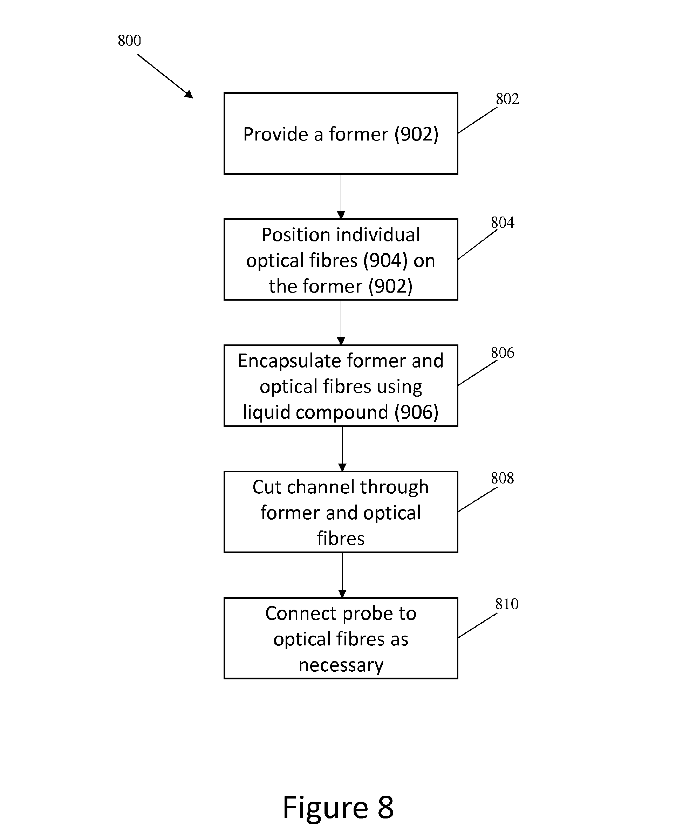

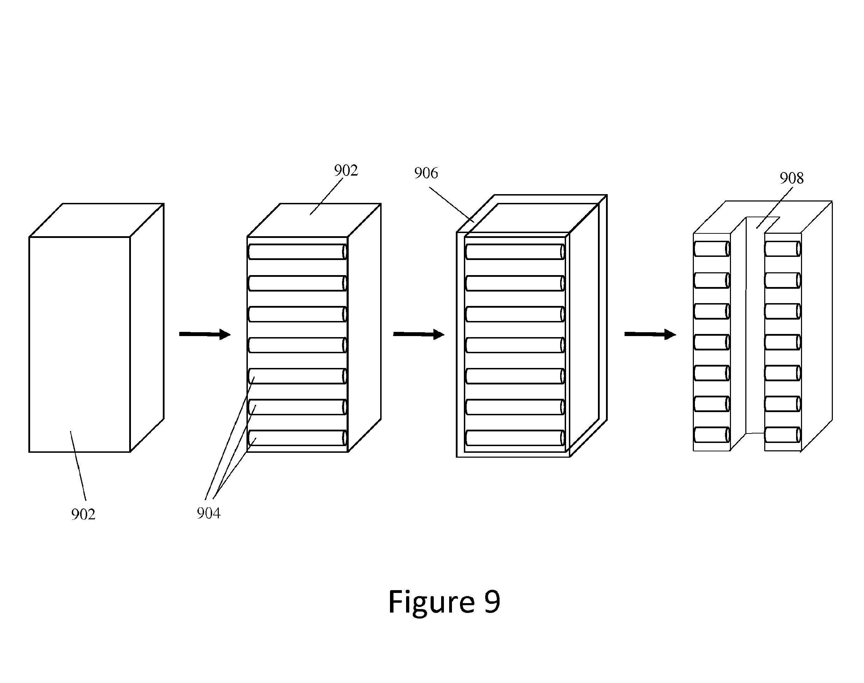

In order to obtain accurate measurements from the probe 118, it is important that the inputs, outputs and optical fibres are connected to the probe at specific locations, and that the separation between adjacent analysis elements is approximately constant over the entire probe. A further aspect of the invention provides a method 800 for manufacturing a probe 118, as will be described with reference to Figures 8 and 9. Figure 8 is a flow diagram of the various steps of the manufacturing method, and Figure 9 shows the probe 118 in various stages of its manufacture.

First, a former 902 is provided (step 802) for supporting the analysis elements, and for acting as a support for attaching the probe to a wall of a fuel tank. The former may be formed of non-conductive material such as, for example, plastics, resins or polymers, or a similar material that is not adversely affected by the contents of a fuel tank. Individual optical fibres 904 are then positioned (step 804) on the former 902, spaced apart as required to achieve the required resolution of for the completed probe 118. It will of course be appreciated that, for a probe 118 having only one analysis element, a single optical fibre 904 will be required in step 804. The former 902 and the optical fibres 904 are then potted or encapsulated (step 806) using a liquid compound 906 allowed to solidify on the former. The liquid compound 906 may be suitable resin or thermosetting polymer which, in its liquid form, can coat the former 902, and which can be allowed to solidify, thereby at least partially encapsulating the former. In one embodiment, the former 902 and optical fibres 904 are dipped into the liquid compound 906, then removed so that the liquid compound is allowed to solidify. In another embodiment the liquid compound 906 is poured or sprayed onto the former 902 and optical fibres 904, then left to solidify. The solidified liquid compound 906 secures the optical fibres 904 to the former 902 in the required position.

A groove or channel 908 is then cut through the former 902 and the optical fibres 904 (step 808) to form the inputs and the outputs of the analysis elements of the probe, and a sampling region 316 between the inputs and outputs. By machining the channel or groove 908 in this way, it is possible to ensure that the sampling region 316 is an equal width along the height of the probe 118 and, therefore, that the separation between the input and the output of each analysis element is approximately consistent. In this way, the inputs and outputs of the analysis elements are accurately aligned.

Finally, the optical fibres of the probe are formed into optical fibre bundles and/or connected as necessary to additional optical fibres in the system (step 810). The probe may then be connected to the other components in the system, such as the photo detector, the processor and the light source.

Thus, the invention provides a probe for use in determining a characteristic of contents of a fuel tank, a system incorporating the probe, a method of determining a characteristic of contents of a fuel tank, and methods of manufacture of the probe.

Although the invention has been described above with reference to one or more preferred embodiments, it will be appreciated that various changes or modifications may be made without departing from the scope of the invention as defined in the appended claims.

## Claims

1. A system for determining at least one characteristic of contents within a fuel tank, comprising:
a probe having a plurality of analysis elements, each analysis element having an input for inputting light to a sampling region to be analysed and an output for outputting light that has passed through the sampling region from the input;
a spectrometer configured to analyse the light received from each analysis element output in order to measure absorption spectra for the contents of the fuel tank; and
a processor configured to compare the measured absorption spectra of the fuel tank contents within the sampling region with known spectra of substances so as to determine at least one substance present within the fuel tank.

2. A system according to claim 1, further comprising a photo detector coupled to the spectrometer, the photo detector configured to convert individual spectral components of the measured absorption spectra into a digital format.

3. A system according to claim 2, wherein the photo detector comprises a charge-coupled device.

4. A system according to any of the preceding claims, further comprising a light source for generating light to be delivered to the input of each analysis element.

5. A system according to claim 4, wherein the light source comprises a broadband light source or a swept narrowband light source.

6. A system according to any of the preceding claims, further comprising a storage medium for storing known absorption spectra of substances.

7. A system according to any of the preceding claims, wherein each analysis element comprises an optical fibre, and the input and the output comprise ends of the optical fibre.

8. A system according to any of the preceding claims, wherein each analysis element further comprises at least one of: an optical element for collecting the light received via the input, an optical element for collecting the light output by the analysis element, an optical element for collimating the light received via the input, and an optical element for collimating the light output by the analysis element.

9. A system according to claim 8, wherein at least one of the optical elements comprises a lens.

10. A system according to any of the preceding claims, wherein the probe is configured to be mounted to an interior wall of an aircraft fuel tank.

11. A system according to any of the preceding claims, wherein the probe is configured to be mounted to a rib within an aircraft wing.

12. A system according to any of the preceding claims, wherein the plurality of analysis elements are arranged linearly.

13. A system according to any of the preceding, wherein the system comprises at least three probes.

14. An aircraft comprising a system according to any of the preceding claims.

15. A method of analysing contents of a fuel tank, the method comprising:
providing, within a fuel tank, a probe having a plurality of analysis elements, each analysis element having an input for inputting light to a sampling region to be analysed and an output for outputting light that has passed through the sampling region from the input;
providing light to each analysis element of the probe via each input;
receiving light from the output of each analysis element;
using a spectrometer, analysing the received light from each analysis element output to measure absorption spectra for the contents of the fuel tank; and comparing the measured absorption spectra of the fuel tank contents within the sampling region with known spectra of substances so as to determine at least one substance present within the fuel tank.
